# EUROPEAN PATENT APPLICATION

(11) **EP 4 751 688 A2**
(43) Date of publication of application: **03.06.2026**
(21) Application number: 26158490.8
(22) Date of filing: 14.09.2023
(51) Int. Cl.: A61F 5/445

(54) **MULTI-LAYER FILM FOR OSTOMY APPLIANCES**

(30) Priority: 22.09.2022 US 202263409047 P
(62) Divisional of application: 23789443.1
(71) Applicant: Hollister Incorporated, Libertyville, IL 60048 (US)
(72) Inventor: ZHOU, Sean, Xixian, Libertyville, 60048 (US)
(74) Representative: FRKelly

(57) **Abstract**

A multilayer film for an ostomy pouch includes a barrier layer that is substantially impermeable to malodor causing compounds and arranged between outer layers. Each of the outer layers is formed from a polymeric mixture comprising about 90 wt.% to about 99 wt.% of a polar polymer and about 1 wt.% to about 10 wt.% of a slip agent and/or an anti-block agent, wherein the polymeric mixture is free of a non-polar polymer.

## Description

### BACKGROUND

The present disclosure relates to a multilayer film material, and more particularly to multi-layer films for ostomy appliances.

Ostomy products and other applications relating to the storage of bodily liquids are highly demanding and typically subject materials used in such products to high levels of moisture. At the same time, it is extremely important that the odor barrier properties of the material are and remain high throughout their useful life. In addition, it is imperative that the mechanical strength of the material is also high and remains high for a sufficiently long period of time for extended use of the product.

Other factors and properties that must also be considered in ostomy product use are the comfort of the material, as such products are worn next to the user's body, the flexibility of the material so that it conforms to the user's movements, and the quietness of the product so that wearing such a product is as audibly imperceptible as possible.

One film used in the manufacture of ostomy pouches, disclosed in International application publication WO93/11938, is a five-layer barrier structure having a gas barrier layer, two moisture barrier layers and optionally one or more adhesive layers disposed therebetween. The moisture barrier layer is a mesophase PP-based material which contacts one or both of the sides of the gas barrier layer. The gas barrier layer is made of an EVOH copolymer.

Other multi-layer films, such as those disclosed in U.S. Patent Nos. 4,239,826 and 4,254,169, which include an oxygen barrier layer formed of vinyl alcohol polymer or copolymers (e.g. PVOH, EVOH), and moisture barrier layers formed of partially hydrolyzed vinyl acetate polymer or copolymer, or modified polyolefins, are also known. Such films have been found to be useful in the manufacture of food packaging containers.

A five layer chlorine-free ostomy pouch film is also known. Such a film, which is disclosed in Giori, U.S. Patent No. 7,270,860 and is incorporated herein in its entirety by reference, has a core odor barrier layer formed of a blend or a compound including amorphous polyamide, and anhydride-modified olefin polymer or copolymer. The film also includes two tie layers on both sides of the core layer and two EVA or EVA-based surface layers.

Still another multilayer film for an ostomy bag is disclosed in Chang et al., U.S. Patent No. 9,301,869 and is incorporated herein in its entirety by reference. Chang et al. discloses a multilayer film including a barrier layer formed from amorphous polyamide resin and a maleic anhydride modified olefin or an epoxy modified olefin, tie layers formed from maleic anhydride (MAH) grafted blend of ethylene propylene rubber (EPR) and polypropylene (PP), inner layers formed from one of an ethylene propylene copolymer based resin, an ethylene-octene based resin and blends-thereof, and outer layers formed from a blend of ethylene vinyl acetate and polypropylene-based resin.

Present disclosure provides a multilayer film having improved sealing properties when compared to multilayer films including outer layers that contain a non-polar polymer, while maintaining other film properties necessary for ostomy appliances.

### BRIEF SUMMARY

In one aspect, a multilayer film for an ostomy pouch may include a barrier layer arranged between first and second outer layers. The barrier layer may be substantially impermeable to malodor causing compounds. Each of the first and second outer layers may be formed from a polymeric mixture comprising about 90 wt.% to about 99 wt.% of a polar polymer, such as acidic copolymer, ester copolymer, maleic anhydride modified polyolefin, and blends thereof, and about 1 wt.% to about 10 wt.% of a slip agent and/or an anti-block agent, wherein the polymeric mixture may be free of a non-polar polymer.

In an embodiment, the multilayer film may be configured to have a kinetic coefficient of friction of about 0.05 to about 0.30 when measured according to ASTM D1894. For example, the multilayer film may be configured to have a kinetic coefficient of friction of about 0.06 to about 0.28 or 0.08 to about 0.25.

In an embodiment, the multilayer film may be configured to have a blocking force below about 100g/100cm² when measured according to ASTM D3354. For example, the multilayer film may be configured to have a blocking force below about 80g/100cm².

In an embodiment, the multilayer film may be configured to have a kinetic coefficient of friction of about 0.06 to about 0.20 when measured according to ASTM D1894 and a blocking force below about 70g/100cm² when measured according to ASTM D3354.

In an embodiment, each of the first and second outer layers may be formed from a polymeric mixture comprising about 92 wt.% to about 97 wt.% of a polar polymer, such as acidic copolymer, ester copolymer, maleic anhydride modified polyolefin, and blends thereof, and about 3 wt.% to about 8 wt.% of a slip agent and/or an anti-block agent. For example, each of the first and second outer layers may be formed from a polymeric mixture comprising about 93 wt.% to about 96 wt.% of a polar polymer, such as acidic copolymer, ester copolymer, maleic anhydride modified polyolefin, and blends thereof, and about 4 wt.% to about 7 wt.% of a slip agent and/or an anti-block agent.

In some embodiments, the polar polymer may be selected from the group consisting of ethylene vinyl acetate (EVA) copolymer, ethylene methyl acrylate copolymer, ethylene acrylic acid copolymer, ethylene methyl acrylic acid copolymer, and polylactic acid homopolymer. The slip agent may be selected from the group consisting of oleamide, erucamide, stearamide, behenamide, oley/palmitamide, stearyl erucamide, ethylene bis-steramide, ethylene bis-oleamide, and the blends thereof. The anti-block agent may be selected from the group consisting of silica, talc, kaolin, mica, calcium carbonate, paraffinic waxes, ethylene and propylene oxide. fatty acid soaps, fatty acid amides, silicones.

In an embodiment, each of the first and second outer layers may be formed from a polymeric mixture comprising about 93 wt.% to about 95 wt.% of an EVA copolymer, about 3 wt.% to about 5 wt.% of a slip agent, and about 1 wt.% to about 3 wt.% of an anti-block agent. In such an embodiment, the multilayer film may be configured to have a kinetic coefficient of friction of about 0.06 to about 0.20 when measured according to ASTM D1894 and a blocking force below about 70g/100cm2 when measured according to ASTM D3354. For example, each of the first and second outer layers may be formed from a polymeric mixture comprising about 94 wt.% of an EVA copolymer, about 4 wt.% of a slip agent, and about 2 wt.% of an anti-block agent, wherein the multilayer film may have a kinetic coefficient of friction of about 0.06 to about 0.20 when measured according to ASTM D1894 and a blocking force below about 70g/100cm2 when measured according to ASTM D3354.

In some embodiments, the barrier layer may be formed from a mixture comprising about 65 wt.% to about 100 wt.% of an amorphous polyamide and about 0 wt.% to about 35 wt.% of a maleic anhydride modified olefin or an epoxy modified olefin.

In an embodiment, the multilayer film may have an outer layer/inner layer/tie layer/barrier layer/tie layer/inner layer/outer layer construction. In such an embodiment, the tie layers may be formed from a maleic anhydride grafted resin, wherein the resin may be one or more of an ethylene-based copolymer, a propylene-based copolymer, an ethylene-octene polymer and a styrene block copolymer. The inner layers may be formed from an ethylene propylene copolymer (polypropylene elastomer) based resin, an ethylene-octene based resin or blends thereof. The multilayer film may be configured to have an Elmendorf tear strength in machine direction measured by ASTM D19222-09 of at least about 200g/mil. In some embodiments, the multilayer film may be symmetrical about the barrier layer.

In an embodiment, an ostomy pouch may include two side walls, wherein each of the side walls may be formed of the multilayer film according to any of the forgoing embodiments. The ostomy pouch may also include a stoma-receiving opening on one of the side walls. In such an embodiment, the two side walls may be heat sealed together along peripheral edges of the side walls.

Other aspects, objectives and advantages will become more apparent from the following detailed description when taken in conjunction with the accompanying drawings.

### BRIEF DESCRIPTION OF THE SEVERAL VIEWS OF THE DRAWINGS

The benefits and advantages of the present embodiments will become more readily apparent to those of ordinary skill in the relevant art after reviewing the following detailed description and accompanying drawings, wherein:
FIG. 1 is a cross-sectional illustration of a multilayer film according to an embodiment;
FIG. 2 is an illustration of a two-piece ostomy pouch system including a pouch formed from a multilayer film and a flange attached thereto according to an embodiment;
FIG. 3 is an illustration of an outlet of a drainable ostomy pouch formed from a multilayer film and closure members attached thereto according to an embodiment;
FIG. 4 is an ostomy pouch formed from a multilayer film including a filter attached thereto according an embodiment; and
FIG. 5 is a drainable ostomy pouch formed from a multilayer film including an outlet tap attached to an outlet of the pouch according to an embodiment.

### DETAILED DESCRIPTION

While the present disclosure is susceptible of embodiment in various forms, there is shown in the drawings and will hereinafter be described a presently preferred embodiment with the understanding that the present disclosure is to be considered an exemplification and is not intended to limit the disclosure to the specific embodiment illustrated.

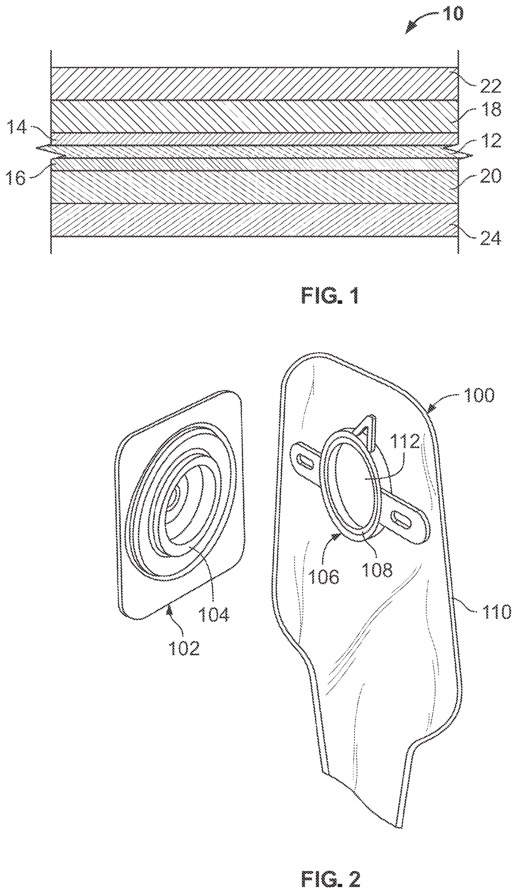

Referring to FIG. 1, a multilayer film 10 may be configured as a seven-layer film comprising a barrier layer 12, tie layers 14, 16, inner layers 18, 20, and outer layers 22, 24. The tie layers 14, 16 may be arranged on each side of the barrier layer 12 and configured to facilitate adhesion of the barrier layer 12 to the remainder of the film structure. The inner layers 18, 20 may be arranged adjacent the tie layers 14, 16 and configured to impart tear strength and "quietness", e.g. low dB(A) level, to the multilayer film 10. Other layers of the multilayer film 10 may also be configured to impart these properties to the multilayer film 10. The outer layers 22, 24 may be arranged adjacent the inner layers 18, 20 and configured to provide heat sealing properties necessary to form an ostomy pouch. In such an embodiment, the multilayer film 10 may have the structure ABCDCBA, where A represents the outer layers, B represents the inner layers, C represents the tie layers, and D represents the barrier layer. In other embodiments, the multilayer film may include more than seven film layers or less than seven film layers. For example, a multilayer film according to this disclosure may be configured to as a six-layer film including a barrier layer, tie layers, an inner layer, and outer layers (i.e. ABCDCA), or a five-layer film including a barrier layer, tie layers, and outer layers (i.e. ACDCA).

### Barrier Layer

Suitable materials for the barrier layer 12 may include amorphous polyamide, anhydride-modified olefinic polymer or copolymer, and an epoxy modified olefin polymer or copolymer. The amorphous polyamide may have a partially aromatic structure and may be produced by condensation of aliphatic diamine with aromatic diacid, or combination of diacids, in molar amounts equivalent to the diamine used. For example, partially aromatic nylons such as 6I/6T, MXDI/61, MXD6/MXDI (wherein I is isophthalic acid, T is terephthalic acid, 6 is hexamethylenediamine, and MXD is metaxylenediamine) are suitable amorphous polyamide. While a variety of amorphous polyamide resins may be used, effective results have been obtained with a polyamide resin marketed as Selar^{®}, such as Selar^{®} PA3426, by DuPont Company. Selar^{®} PA3426 is understood to be substantially amorphous with a density of about 1.19 grams per cubic centimeter (g/cc) and a glass transition temperature (dry) of about 127°C. It has a relatively high melt strength and can be used under a broader range of processing conditions than conventional crystalline nylons.

Other suitable amorphous nylons may include Grivory^{®}, such as Grivory^{®} G21 and Grivory^{®} HB5299, which are commercially available from EMS-Chemie of Sumter, SC. Grivory^{®} G21 has a density of about 1.18 grams per cubic centimeter (g/cc) and a glass transition temperature (dry) of about 128°C. Grivory^{®} HB5299 has a density of about 1.2 g/cc and a glass transition temperature (dry) of about 95°C and a melting point temperature of about 219°C.

In some embodiments, the barrier layer 12 may be formed from amorphous polyamide resin compounded or blended with maleic anhydride grafted blend of ethylene propylene rubber (EPR) and polypropylene (PP) (e.g. Zelas^{®} MC721 AP from Mitsubishi), or maleic anhydride grafted or copolymerized ethylene methyl acrylate (EMA), ethylene vinyl acetate (EVA), ethylene butyl acrylate (EBA), ethylene ethyl acrylate (EEA) or other polyolefins (e.g. Lotader^{®} 4720 from Arkema, Bynel^{®} from DuPont, Plexar^{®} form Lyondellbassell), or glycidyl methacrylate (GMA) grafted polyethylene (PE), EMA, or other polyolefins (e.g. Lotader^{®} from Arkema), or maleic anhydride modified styrene-ethylene-butylene-styrene (SEBS) copolymer or other styrene block copolymers. In an embodiment, the barrier layer 12 may be formed from a blend of amorphous polyamide and anhydride-modified olefin polymer or copolymer. For example, the barrier layer 12 may be formed from a blend comprising about 65 % wt.% to about 100 wt.%, preferably about 75 wt.% to about 95 wt.% of amorphous polyamide resin, and about 0 wt.% to about 35 wt.%, preferably about 5 wt.% to about 25 wt.% of anhydride-modified olefinic polymer or copolymer.

In an embodiment, the anhydride-modified olefinic polymer or copolymer may be a copolymer of ethylene and at least one ester containing comonomer, or a blend thereof, modified (grafted or copolymerized) with about 0.01 wt.% to about 2 wt.% of unsaturated carboxylic anhydride (serving as a compatibilizing agent). In another embodiment, the anhydride-modified olefinic polymer or copolymer may be a copolymer of ethylene and an alpha-olefin, or a blend thereof, grafted or copolymerized with about 0.01 wt. % to about 2 wt.% of unsaturated carboxylic anhydride. The olefinic polymer or copolymers may be functionalized by copolymerization or grafting with unsaturated carboxylic anhydride, such as maleic anhydride. The level of maleic anhydride needed to functionalize the olefinic polymer may be quite low, for example, less than about 2 wt.%.

In an embodiment, the anhydride-modified copolymer may be formed from ethylene and at least one ester-containing comonomer, or a blend thereof, grafted or copolymerized with about 0.01 wt.% to about 2 wt.% of the unsaturated carboxylic anhydride, wherein the anhydride content may be under about 0.5 wt.%. The ester-containing comonomer may be alkylacrylate, preferably ethylacrylate. One such copolymer is available from Arkema, Inc., of France, under the tradename Lotader^{®} 4720. Lotader^{®} 4720 is ethylene-ethyl acrylate-maleic anhydride terpolymer having a density of 0.944 g/cc, wherein the ethyl acrylate content is about 30 wt.% and the maleic anhydride content is about 0.3 wt. %. Another suitable maleic anhydride grafted copolymer is ethylene methyl acrylate-maleic anhydride polymer available from Arkema under the tradename Lotader^{®} 4603.

Similar performance may be achieved with other anhydride-modified olefinic polymers or copolymers sharing comparable low density, such as ethylene-propylene copolymers, ethylene methyl acrylate copolymers, and terpolymer (EPM, EMA and EPDM). EPM and EPDM have a density in the range of 0.85 to 0.86 g/cc and may be suitable for modification with maleic anhydride.

In an embodiment, the barrier layer 12 may be formed from a blend of amorphous polyamide and maleic anhydride (MAH) grafted styrene-ethylene-butylene-styrene (SEBS) copolymer, wherein the copolymer may include about 1.0 wt. % of maleic anhydride. For example, the barrier layer 12 may be formed from a blend comprising about 85 wt. % of amorphous nylon and about 15 wt. % of MAH grafted SEBS copolymer, which may be configured to provide desirable malodor blocking and tear strength properties for an ostomy appliance. An example of suitable MAH grafted SEBS copolymers is Kraton^{®} FG1924, commercially available from Kraton Polymers US, LLC.

In some embodiments, functional groups other than MAH may be used in the barrier layer resins. For example, epoxy functional rubber can be used, such as glycidyl methacrylate (GMA) copolymerized with ethylene and other monomers. One such resin is ethylene-methyl acrylate glycidyl methacrylate (E-MA-GMA), available from Arkema as Lotader^{®} AX8920, and ethylene- glycidyl methacrylate (E-GMA), also available from Arkema as Lotader^{®}AX8840.

### Tie Layers

Each of the tie layers 14, 16 may be formed from a same material or different materials. Suitable materials for the tie layers 14, 16 may include, but are not limited to, maleic anhydride (MAH) grafted blend of ethylene propylene rubber (EPR) and polypropylene (PP) (e.g. Zelas^{®} MC787AP from Mitsubishi), MAH grafted or copolymerized EMA, EVA, EBA, EEA or other polyolefins (e.g. Lotader^{®} from Arkema, Bynel^{®} from DuPont, Plexar^{®} from Lyondellbassell), MAH grafted polypropylene (PP) concentrate (e.g. Bynel^{®} from DuPont) blend with ethylene-propylene copolymer (PP-elastomer) (e.g. Vistamaxx^{®} from Exxon, Versify^{®} from Dow), ethylene-octene (EO) plastomer (e.g. Exact^{®} from Exxon, Affinity^{®} from Dow), EMA (e.g. Lotryl^{®} from Arkema), or other polyolefins, or GMA grafted PE, EMA or other polyolefins (e.g. Lotader^{®} from Arkema.) In an embodiment, one or both of the tie layers 14, 16 may be formed from olefin-based thermoplastic elastomer (EPR rubber), or MAH grafted EMA copolymers, or blends of EMA and MAH grafted linear low density polyethylene (LLDPE), PE, EVA copolymers, or ethylene modified with functional anhydride groups.

In an embodiment, one or both of the tie layers 14, 16 may be formed from a blend of about 80 wt.% of ethylene methyl acrylate (EMA) copolymer comprising methyl acrylate at about 18.5 wt.% of the EMA copolymer, and about 20 wt.% of MAH grafted linear low density polyethylene (LLDPE) comprising maleic anhydride at about 0.8 wt.% to 1.1 wt.% of the MAH - LLDPE polymer. A suitable example of the EMA copolymer is available from Arkema, Inc. under the tradename Lotryl^{®}18MA02, which has a melting point temperature of 87°C and a Shore hardness of 25. A suitable example of the MAH grafted LLDPE polymer is available from DuPont Company under the tradename Bynel^{®}CXA41E710.

In another embodiment, one or both of the tie layers 14, 16 may be formed from MAH grafted EMA copolymer comprising maleic anhydride present at about 0.3 wt.% and methyl acrylate present at about 20 wt.% of the copolymer, such as the copolymer available under the tradename Lotader^{®}4503 from Arkema, Inc. having a melting point temperature of 78°C and a Shore D hardness of 25.

In yet another embodiment, one or both of the tie layers 14, 16 may be formed from MAH grafted blend of EPR and PP, such as the resin available under the tradename Zelas^{®} MC787AP from Mitsubishi Chemical Co. This resin has a melting point temperature of 158°C, a Shore A hardness of 75 and a specific gravity of 0.89. In such an embodiment, the tie layers 14, 16 may be configured to securely attach the barrier layer 12 to the adjacent film layers and provide a multilayer film 10 having improved mechanical strength.

In an embodiment, one or both of the tie layers 14, 16 may be formed from epoxy functional rubber, such as glycidyl methacrylate (GMA) copolymerized with ethylene and other monomers, such as E-MA-GMA (e.g. Lotader^{®}AX8920) and E-GMA (e.g. Lotader^{®}AX8840).

### Inner layers

Each of the first and second inner layers, 18, 20 may be formed from a same material or different materials. The inner layers 18, 20 may be configured to impart mechanical (tear) strength and quietness to the multilayer film 10. Suitable materials for the inner layers 18, 20 may include ethylene based polymers, such as ethylene vinyl acetate (EVA) copolymer, ethylene-octene (EO) plastomers, and ethylene-propylene (EP) copolymers (PP-elastomer). and propylene based elastomers, and thermoplastic olefins. In an embodiment, one or both of the inner layers 18, 20 may be formed from ethylene vinyl acetate (EVA) copolymer comprising vinyl acetate at about 8 wt.% to 30 wt.%, preferably about 10 wt.% to about 25 wt.% of the copolymer, such as the EVA copolymer available under the tradename Escorene^{®}FL00218 from ExxonMobil Corporation, which has a melting point temperature of about 86°C and Shore A hardness of about 91.

In another embodiment, one or both of the inner layers 18, 20 may be formed from EO plastomer, such as the resin available under the tradename Exact^{®} 0203 from ExxonMobil Corporation, which has a specific gravity of about 0.88 and Shore A hardness of about 95. In yet another embodiment, one or both of the inner layers 18, 20 may be formed from ethylene-propylene copolymer (PP-elastomer), such as the PP-elastomer resin available under the tradename Versify^{®}2200 from Dow Chemical, which has a melting point of about 82°C, Shore A hardness of 94, Shore D hardness of 42, and specific gravity of 0.878.

In some embodiments, one or both of the inner layers 18, 20 may be formed from a blend of various PP copolymer resins. For example, one or both of the inner layers 18, 20 may be formed from a blend of Versify^{®}2200 and Versify^{®}3400, wherein the blend may be formulated to include about 50 wt.% to about 75 wt.% of Versify^{®}2200. A suitable blend may be formulated to have a melting point of about 97°C, Shore A hardness of about 72, Shore D hardness of about 22, and specific gravity of about 0.865. In an embodiment, one or both of the inner layers 18, 20 may be formed from PP-elastomers such as Versify^{®} from Dow, Vistamaxx^{®} from Exxon, and Notio^{®} from Mitsui, or propylene ethylene copolymer such as Adflex^{®} Q100F.

### Outer Layers

In an embodiment, the outer layers 22, 24 may be configured to provide improved sealing properties when compared to films comprising a non-polar polymer, such as polypropylene polymer or copolymers, in their outer layers. FIG. 2 shows a two-piece ostomy pouch system including an ostomy pouch 100 and a skin barrier appliance 102 according to an embodiment. The skin barrier appliance 102 may be configured for attachment to user's peristomal skin and include a body-side coupling member 104. The ostomy pouch 100 may include a flange 106 comprising a pouch-side coupling member 108, which may be configured to engage with the body-side coupling member 104 to attach the ostomy pouch 100 to the skin barrier appliance 102. The ostomy pouch 100 may be formed from the multilayer film 10. For example, two sheets of the multilayer film 10 may be heat or otherwise sealed around their peripheries to form a peripheral seal 110, wherein a collection chamber for collecting body waste may be defined therebetween. The ostomy pouch 100 may include an inlet opening 112 for receiving body waste, and the flange 106 may be attached, for example, via heat sealing, to the ostomy pouch 100 surrounding the inlet opening 112. In such an embodiment, the outer layers 22, 24 of the multilayer film 10 may be sealed together to form the peripheral seal 110, and/or sealed to the flange 106.

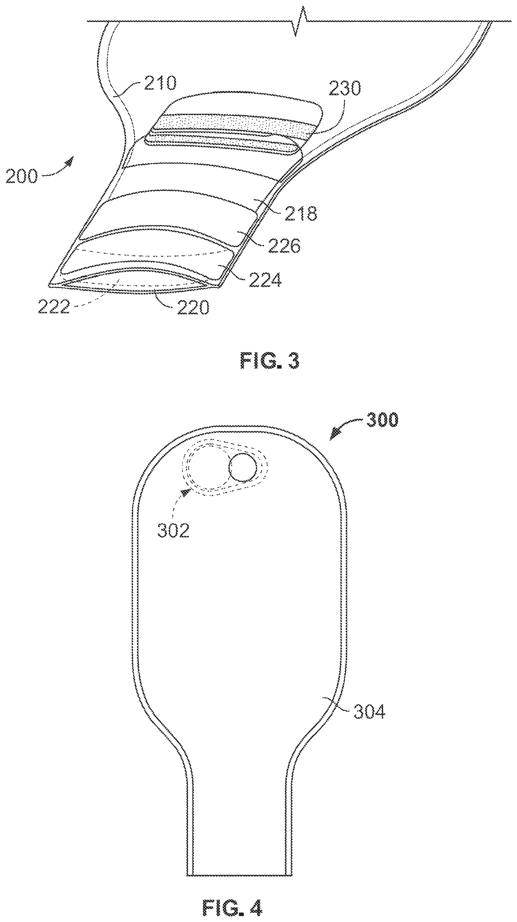

In an embodiment, a drainable ostomy pouch 200 may be formed from the multilayer film 10 and include a plurality of closure members 222, 224, 226 as shown in FIG. 3. The plurality of closure members 222, 224, 226 may be attached to an outlet portion 218 of the drainable ostomy pouch 200, wherein an outlet opening 220 for discharging body waste collected in the pouch 200 is defined. The drainable ostomy pouch 200 may be configured to be closed by rolling up the outlet portion 218 and securing it with a pair of fastening members 230. The plurality of closure members 222, 224, 226 may be configured to facilitate and provide liquid-tight closure of the outlet portion 218. In such an embodiment, the outer layers 22, 24 of the multilayer film 10 may be heat sealed to form a peripheral seal 210, and the plurality of closure members 222, 224, 226 may be attached to the outer layers 22, 24, for example, via an adhesive.

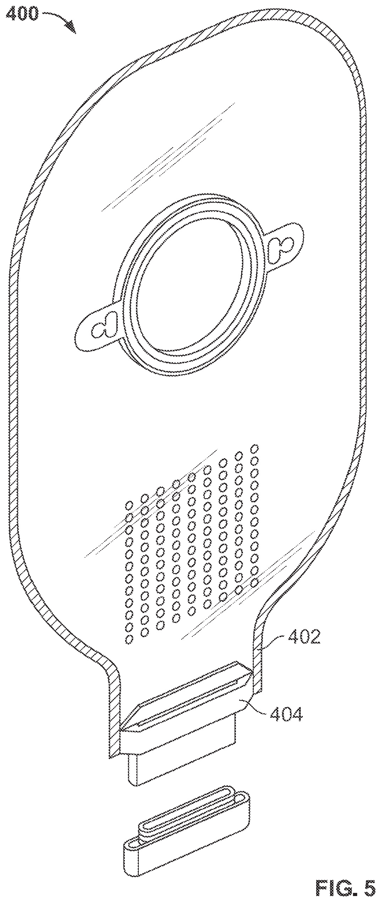

In FIG. 4, an ostomy pouch 300 may be formed from the multilayer film 10 and include a filter 302 attached to a distal side 304 of the pouch 300 according to an embodiment. In FIG. 5, an ostomy pouch 400 may be formed from the multilayer film 10 and include an outlet tap 404 attached to an outlet portion 402 of the pouch 400 according to an embodiment.

The outer layers 22, 24 of the multilayer film 10 may be formed from a same or different materials. In an embodiment, the outer layers 22, 24 may be configured to improve chemical compatibility of the multilayer film 10 with other components that are sealed or adhered to an ostomy pouch formed from the multilayer film 10. For example, the outer layers 22, 24 may be configured to provide excellent sealing strength and adhesion strength when two sheets of the multilayer film 10 are heat sealed along their peripheries to form a peripheral seal 110, 210, or when a filter 302 or an outlet tap 404 are heat sealed to an ostomy pouch 300, 400, or when the plurality of closure members 222, 224, 226 are attached to the ostomy pouch 100, 200. While providing good sealing properties and adhesion properties, the outer layers 22, 24 may also be configured to have a relatively low coefficient of friction and blocking force to facilitate processing of the multilayer film 10 using pouch machines and to facilitate discharging of body waste collected in the drainable ostomy pouch 100, 200 formed from the multilayer film 10.

In such an embodiment, the outer layers 22, 24 may be formed from a polymeric compound that is free of non-polar polymer. For example, the polymeric compound may be formulated with a polar polymer, such as acidic copolymer, ester copolymer, maleic anhydride modified polyolefin, and blends thereof, slip agent and anti-block agent. The polymeric compound, which does not contain any non-polar polymer, may be configured to provide the outer layers 22, 24 having improved heat sealing properties when compared to those containing a non-polar polymer, such as polypropylene polymers and copolymers.

In an embodiment, the outer layers 22, 24 may be formed from a polymeric compound that is free of a non-polar polymer and comprising about 90 wt.% to about 99 wt.% a polar polymer, such as acidic copolymer, ester copolymer, maleic anhydride modified polyolefin, and blends thereof, preferably about 92 wt.% to about 97 wt.%, more preferably about 93 wt.% to about 96 wt.%, and about 1 wt.% to about 10 wt.% functional additives and fillers including a slip agent and anti-block agent, preferably about 3 wt.% to about 8 wt.%, more preferably about 4 wt.% to about 7 wt.%. The outer layers 22, 24 may have a kinetic coefficient of friction of about 0.05 to about 0.30, preferably about 0.06 to about 0.28, and more preferably about 0.08 to about 0.25 when measured according to ASTM D1894, and a blocking force of below about 100g/100cm² (e.g. about 1g/100cm² to about 100g/100cm²), preferably below 90g/100cm² (e.g. about 1g/100cm² to about 90g/100cm²), more preferably below 80g/100cm² (e.g. about 1g/100cm² to about 80g/100cm²) when measured according to ASTM D3354.

Suitable polar polymers for the outer layers 22, 24 may include ethylene vinyl acetate (EVA) copolymer, ethylene methyl acrylate (EMA) copolymer, ethylene acrylic acid copolymer, ethylene methyl acrylic acid copolymer, and polylactic acid homopolymer. EVA copolymer may contain about 10 wt.% to 35 wt.% vinyl acetate, preferably about 18 wt.% vinyl acetate. Suitable EVA copolymers may include Escorene^{®} Ultra FL00218 available from ExxonMobil, which has a melting point temperature of about 86°C and Shore A hardness of about 91. EMA copolymer may include about 10 wt.% to about 35 wt.% of methyl acrylate, preferably about 18.5 wt.% to about 30 wt.%. Suitable EMA copolymers may include Lotryl^{®}18AM02 available from Arkema Inc. and Elvaloy^{®}1330AC available from DuPont, which may have a melting point temperature of about 85°C to about 87°C, and Shore A hardness of about 73, and Shore D hardness of about 20 to 25.

Suitable slip agents for the outer layers 22, 24 may include fatty acid amides, such as oleamide, erucamide, stearamide, behenamide, oley/palmitamide, stearyl erucamide, ethylene bis-steramide, ethylene bis-oleamide, and the blends thereof. Examples of the suitable slip agent may include Polybatch^{®} T02569 from A. Schulman Inc. Suitable anti-block agents for the outer layers 22, 24 may include silica, talc, kaolin, mica, calcium carbonate, paraffinic waxes, ethylene and propylene oxide, fatty acid soaps, fatty acid amides, silicones. Examples of the suitable anti-block agent may include Polybatch^{®} SAS from A. Schulman Inc.

In an embodiment, the outer layers 22, 24 may be formed from a compound comprising about 93 wt.% to about 95 wt.% of EVA copolymer, such as Escorene^{®} Ultra FL00218, and about 3 wt.% to about 5 wt.% of slip agent, such as Polybatch^{®} T02569, and about 1 wt.% to about 3 wt.% of anti-block agent, such as Polybatch^{®} SAS. wherein the compound is free of non-polar polymer. For example, the outer layers 22, 24 may be formed from a compound comprising about 94 wt.% of EVA copolymer, such as Escorene^{®} Ultra FL00218, and about 4 wt.% of slip agent, such as Polybatch^{®} T02569, and about 2 wt.% of anti-block agent, such as Polybatch^{®} SAS. In an embodiment, a multilayer film may include a barrier layer 12 and outer layers 22, 24 formed from the non-polar polymer free compound.

In an embodiment, the outer layers 22, 24 may be formed from a compound comprising about 95.5 wt.% of EVA copolymer (Escorene^{®} Ultra FL00218), about 3 wt.% of slip/anti block agent (Polybatch^{®} SAB1982VA), and about 1.5 wt.% of polymer processing aid, AMF705HF). In another embodiment, the outer layers 22, 24 may be formed from a compound comprising about 96 wt.% of EVA copolymer (Escorene^{®} Ultra FL00218), about 3 wt.% of slip agent (Polybatch^{®} T02569), and about 1 wt.% of anti-block agent (Polybatch^{®} M65). In yet another embodiment, the outer layers 22, 24 may be formed from a compound comprising about 95 wt.% of EVA copolymer (Escorene^{®} Ultra FL00218), about 4 wt.% of slip agent (Polybatch^{®} T02569), and about 1 wt.% of anti-block agent (Polybatch^{®} M65). In an embodiment, the outer layers 22, 24 may be formed from a compound comprising about 94 wt.% of EVA copolymer (Escorene^{®} Ultra FL00218), about 4 wt.% of slip agent (Polybatch^{®} T02569), and about 2 wt.% of anti-block agent (Polybatch^{®} M65).

### Multilayer Film

In some embodiments, the multilayer film 10 may be configured as a symmetrical film. In such an embodiment, the configuration of the layers on opposing sides of the barrier layer 12, namely the tie layers 14, 16, inner layers 18, 20 and outer layers 22, 24 may be identical. In an embodiment, the barrier layer 12 may have a thickness of about 2 microns (µm) to about 6 µm; each of the tie layers 14, 16 may have a thickness of about 2µm to about 6µm; each of the inner layers 18, 20 may have a thickness of about 6µm to about 24µm; and the outer layers 22, 24 may have a thickness of about 6µm to about 30µm. Accordingly, the multilayer film 10 may have a total thickness of about 30µm to about 126µm. In an embodiment, the multilayer film 10 may include the barrier layer 12 having a thickness between about 3µm and 5µm, preferably about 4µm; the tie layers 14, 16, each tie layer having a thickness between about 2µm and 5µm, preferably between about 3µm and 4µm; the inner layers 18, 20, each inner layer having a thickness between about 10µm and about 17µm, preferably between about 13µm and about 15µm; and the skin/seal layers 22, 24, each skin/seal layer having a thickness between about 12µm and about 22µm, preferably between about 18µm and about 19µm. Thus, the film 10 of this embodiment has a thickness between about 51µm and about 93µm, preferably between about 72µm and about 80µm.

The multilayer film 10 may be formed by a coextruding process, wherein the different thermoplastic resins for the barrier layer 12, two tie layers 14, 16, inner layers 18, 20 and outer layers 22, 24 may be fed continuously into respective extruders, melted in the extruders and transported from a feed-block or combining adaptor into a die where the different polymers, one layer over and adhering to the other, exit the die slot. Such a coextrusion process or another process for forming such a film will be recognized by those skilled in the art.

In another embodiment, the multilayer film 10 may be formed by a lamination process. In yet another embodiment, one of more layers of the multilayer film 10 may be applied via a coating process, such as an extrusion coating process.

### Examples and Test Results

Several different multilayer film samples including outer layers containing a non-polar polymer and outer layers that are free of a non-polar polymer were prepared and tested. Group 1 film samples included outer layers formed from a mixture comprising EVA polymer (e.g. Escorene^{®} FL00218) and PP elastomer (e.g. Vistamaxx^{®} 3980FL). Group 2 film samples included outer layers formed from a mixture comprising EVA polymer (Escorene^{®} FL00218) and free of a non-polar polymer. The film samples were heat sealed to 2.25 inches gaskets using an impulse sealer equipped with a wedged sealer tip which controlled seal depth to mimic a laminate seal as closely as possible. The sealing conditions were 365°F, 1.5 seconds and 50 psi. Samples were prepared by cutting a ¼"-wide piece of the heat seal at the 12 o'clock position of gasket, then tested under tension mode using a MTS Tensile Tester. Seal strength, elongation and failure mode were measured.

**TABLE 1 Seal Strength, Elongation and Failure Mode of Film Samples to Gaskets**

| **Film Samples** | **Strength, lb/in** | | |
|---|---|---|---|
| | **Dow 722 NI Gasket** | **Escorene Gasket** | **Dual Blend Gasket** |
| **Group 1** | 4.3 | 5.5 | 7.2 |
| **Group 2** | 4.4 | 6.7 | 8.1 |

| | **Elongation, inches** | | |
|---|---|---|---|
| | **Dow 722 NI Gasket** | **Escorene Gasket** | **Dual Blend Gasket** |
| **Group 1** | 0.823 | 4.158 | 5.605 |
| **Group 2** | 1.73 | 5.248 | 6.127 |

| | **Failure Mode** | | |
|---|---|---|---|
| | **Dow 722 NI Gasket** | **Escorene Gasket** | **Dual Blend Gasket** |
| **Group 1** | seal peeled | seal peeled/1 tore | seal peeled |
| **Group 2** | seal peeled | Tore at Seal/1 peel | Tore at Seal/1 peel |

As shown in Table 1, Group 2 film samples, which included outer layers that are free of a non-polar polymer, exhibited improved seal strength, elongation, and the failure mode when compared to Group 1 film samples, which included outer layers containing a non-polar polymer.

Heat seal strength between seven-layer film samples and filter packet films was evaluated by pull testing after heat seal formation. Each of the seven-layer film samples were heat sealed to two different filter packet films using an impulse bar sealer set to 140°C, 160°C and 170°C. The sealing pressure was 50 psi and sealing time was 1 second. The sealed samples were then cut across the seal such that each side of the seal had a 1" wide by 3" long tail to mount into the test fixture. Seal strength was tested under tension mode using a MTS Tensile Tester.

**TABLE 2 Seal Strength, Elongation and Failure Mode of Film Samples to Filter Packet Films**

| **Film Samples** | **Seal Strength, lbf (Film Sample to Filter Packet Film Seal)** | | | | | |
|---|---|---|---|---|---|---|
| | **P1341-2** | | | **P1341-6** | | |
| | 140°C | 160°C | 170°C | 140°C | 160°C | 170°C |
| XT1336-1 | 4.7 | 4.5 | 4.5 | 4.8 | 4.9 | 4.8 |
| XT1336-2 | 7.0 | 7.0 | 6.9 | 4.9 | 5.2 | 4.9 |
| XT 1336-3 | 7.1 | 7.0 | 7.0 | 5.0 | 5.1 | 5.0 |
| XT1336-4 | 7.1 | 7.1 | 6.8 | 5.0 | 5.1 | 5.0 |
| XT1336-5 | 7.2 | 5.7 | 6.9 | 5.0 | 5.0 | 5.1 |
| XT 1336-6 | 7.2 | 7.0 | 7.1 | 5.0 | 5.0 | 5.0 |

| | **Elongation, % (Film Sample to Filter Packet Film Seal)** | | | | | |
|---|---|---|---|---|---|---|
| | **P1341-2** | | | **P1341-6** | | |
| | 140°C | 160°C | 140°C | 160°C | 140°C | 160°C |
| XT1336-1 | 2.0 | 2.0 | 2.2 | 1.9 | 2.1 | 2.2 |
| XT1336-2 | 2.0 | 2.0 | 1.8 | 1.7 | 2.0 | 1.9 |
| XT 1336-3 | 2.5 | 2.1 | 2.0 | 1.9 | 2.1 | 2.0 |
| XT1336-4 | 2.3 | 2.2 | 1.8 | 1.8 | 1.9 | 2.0 |
| XT1336-5 | 2.0 | 1.6 | 1.9 | 1.7 | 2.1 | 2.0 |
| XT1336-6 | 2.0 | 2.1 | 2.0 | 1.8 | 2.0 | 2.0 |

| | **Failure Mode (Film Sample to Filter Packet Film Seal)** | | | | | |
|---|---|---|---|---|---|---|
| | **P1341-2** | | | **P1341-6** | | |
| | 140°C | 160°C | 170°C | 140°C | 160°C | 170°C |
| XT1336-1 | Seal Peeled | Seal Peeled | Seal Peeled | Seal Peeled | Seal Peeled | Seal Peeled |
| XT1336-2 | Tore at Seal | Tore at Seal | Tore at Seal | Seal Peeled | Tore at Seal | Tore at Seal |
| XT1336-3 | Tore at Seal | Tore at Seal | Tore at Seal | Seal Peeled | Tore at Seal | Tore at Seal |
| XT1336-4 | Partial Peel | Tore at Seal | Tore at Seal | Seal Peeled | Tore at Seal | Tore at Seal |
| XT1336-5 | Tore at Seal | Seal Peeled | Tore at Seal | Seal Peeled | Seal Peeled | Tore at Seal |
| XT1336-6 | Tore at Seal | Tore at Seal | Tore at Seal | Seal Peeled | Tore at Seal | Tore at Seal |

XT1336-1 included a barrier layer formed from a mixture comprising about 85 wt.% amorphous polyamide (Selar^{®} PA3426R) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP2C), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 48.5 wt.% EVA polymer (Escorene^{®} FL00218), about 48.5 wt.% PP elastomer (Vistamaxx^{®} 3980FL), about 2 wt.% slip/anti-block agent (Polybatch^{®} SAB1982VA) and about 1 wt.% polymer processing aid (AMF705HF).

XT1336-2 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721 AP2C), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 97 wt.% EVA polymer (Escorene^{®} FL00218), about 3 wt.% of slip/anti block agent (Polybatch^{®} SAB1982VA).

XT1336-3 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP2C), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 96 wt.% of EVA copolymer (Escorene^{®} Ultra FL00218), about 3 wt.% of slip agent (Polybatch^{®} T02569), and about 1 wt.% of anti-block agent (Polybatch^{®} MA65).

XT1336-4 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP2C), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 95 wt.% of EVA copolymer (Escorene^{®} Ultra FL00218), about 4 wt.% of slip agent (Polybatch^{®} T02569), and about 1 wt.% of anti-block agent (Polybatch^{®} MA66).

XT1336-5 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP2C), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 94 wt.% of EVA copolymer (Escorene^{®} Ultra FL00218), about 4 wt.% of slip agent (Polybatch^{®} T02569), and about 2 wt.% of anti-block agent (Polybatch^{®} SAS).

XT1336-6 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP2C), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 96.5 wt.% of EVA copolymer (Escorene^{®} Ultra FL00218), about 2 wt.% of slip agent (Polybatch^{®} T02569), and about 1.5 wt.% of slip/anti block agent (Polybatch^{®} SAB1982VA).

Filter packet film sample P1341-2 included a seal layer formed from EMA copolymer (Nucrel^{®} from DuPont), a core layer formed from medium-density polyethylene (MDPE), a back layer formed from EVA copolymer comprising vinyl acetate at about 4 wt.%. Filter packet film sample P1341-6 included a seal layer formed from a blend of 50 wt.% EAA copolymer (Lotryl^{®} from Dupont) and 50 wt.% LDPE (Dow 722), a core layer formed from medium-density polyethylene (MDPE), a back layer formed from EVA copolymer comprising vinyl acetate at about 4 wt.%. A seven-layer film sample and a filter packet film were heat sealed together to form a seal between a seal layer of the filter packet film and an outer layer of the seven-layer film sample.

As shown in Table 2, the seven-layer film samples XT1336-2, XT1336-3, XT1336-4, XT1336-5, and XT1336-6, which included outer layers that are free of a non-polar polymer, exhibited improved seal strength, elongation, and the failure mode when compared to the seven-layer film sample XT1336-1, which included outer layers containing a non-polar polymer.

Seven-layer film samples including outer layers formed from a mixture comprising EVA polymer, PP elastomer, and Polybatch^{®} SAB1982VA slip/anti-block agent, and seven-layer film samples including outer layers formed from a mixture that is free of a non-polar polymer and comprising EVA polymer and Polybatch^{®} SAB1982VA slip/anti-block agent were prepared and tested for kinetic coefficient of friction (CoF) according to ASTM D1894. Each of the film samples was configured to have an outer layer/inner layer/tie layer/barrier layer/tie layer/inner layer/outer layer configuration as shown in the embodiment of FIG. 1.

**TABLE 3 Kinetic Coefficient of Friction of Seven-layer Film Samples**

| | **Outer layers** | **Inner layers** | **Tie layers** | **Barrier layer** | **kinetic CoF** | |
|---|---|---|---|---|---|---|
| | | | | | **mean** | **St Dev** |
| **Sample 1** | 48.5wt.% Escorene^{®} FL00218, 48.5wt.% Vistamaxx^{®} 3980FL, 2wt.% Polybatch^{®} SAB1982VA, 1wt.% AMF705HF | 65wt.% Vistamaxx^{®} 3980FL, 35wt.% Adflex^{®} Q100F | Zelas MC721 AP2C | 85wt.% Selar^{®} PA3426R, 15wt.% Lotader^{®} 4720 | 0.133 | 0.0086 |
| **Sample 2** | 48.5wt.% Escorene^{®} FL00218, 48.5wt.% Vistamaxx^{®} 3980FL, 2wt.% Polybatch^{®} SAB1982VA, 1wt.% AMF705HF | 55wt.% Vistamaxx^{®} 3980FL, 35wt.% Adflex^{®} Q100F. 10wt.% Polybatch^{®} T93900 | Zelas MC721 AP2C | 85wt.% Selar^{®} PA3426R, 15wt.% Lotader^{®} 4720 | 0.146 | 0.0010 |
| **Sample 3** | 97wt.% Escorene^{®} FL00218, 2wt.% Polybatch^{®} SAB1982VA, 1wt.% AMF705HF | 65wt.% Vistamaxx^{®} 3980FL, 35wt.% Adflex^{®} Q100F | Zelas MC721 AP2C | 85wt.% Selar^{®} PA3426R, 15wt.% Lotader^{®} 4720 | 0.416 | 0.1285 |
| **Sample 4** | 96wt.% Escorene^{®} FL00218, 3wt.% Polybatch^{®} SAB1982VA, 1wt.% AMF705HF | 65wt.% Vistamaxx^{®} 3980FL, 35wt.% Adflex^{®} Q100F | Zelas MC721 AP2C | 85wt.% Selar^{®} PA3426R, 15wt.% Lotader^{®} 4720 | 0.154 | 0.0100 |
| **Sample 5** | 95wt.% Escorene^{®} FL00218, 4wt.% Polybatch^{®} SAB1982VA, 1wt.% AMF705HF | 65wt.% Vistamaxx^{®} 3980FL. 35wt.% Adflex^{®} Q100F | Zelas MC721 AP2C | 85wt.% Selar^{®} PA3426R, 15wt.% Lotader^{®} 4720 | 0.150 | 0.0155 |
| **Sample 6** | 97wt.% Escorene^{®} FL00218, 2wt.% Polybatch^{®} SAB1982VA, 1wt.% AMF705HF | 55wt.% Vistamaxx^{®} 3980FL, 35wt.% Adflex^{®} Q100F, 10wt.% Polybatch^{®} T93900 | Zelas MC721 AP2C | 85wt.% Selar^{®} PA3426R, 15wt.% Lotader^{®} 4720 | 0.259 | 0.1235 |
| **Sample 7** | 96wt.% Escorene^{®} FL00218, 3wt.% Polybatch^{®} SAB1982VA, 1wt.% AMF705HF | 55wt.% Vistamaxx^{®} 3980FL, 35wt.% Adflex^{®} Q100F, 10wt.% Polybatch^{®} T93900 | Zelas MC721 AP2C | 85wt.% Selar^{®} PA3426R, 15wt.% Lotader^{®} 4720 | 0.187 | 0.0135 |
| **Sample 8** | 95wt.% Escorene^{®} FL00218, 4wt.% Polybatch^{®} SAB1982VA, 1wt.% AMF705HF | 55wt.% Vistamaxx^{®} 3980FL, 35wt.% Adflex^{®} Q100F, 10wt.% Polybatch^{®} T93900 | Zelas MC721 AP2C | 85wt.% Selar^{®} PA3426R, 15wt.% Lotader^{®} 4720 | 0.167 | 0.0283 |

Sample 1 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 48.5 wt.% EVA polymer (Escorene^{®} FL00218), about 48.5 wt.% PP elastomer (Vistamaxx^{®} 3980FL), about 2 wt.% slip/anti-block agent (Polybatch^{®} SAB1982V A) and about 1 wt.% polymer processing aid (AMF705HF).

Sample 2 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat MAH grafted blend of EPR and PP (Zelas^{®} MC721AP), inner layers formed from a mixture comprising about 55 wt.% PP elastomer (Vistamaxx^{®} 3980FL), about 35 wt.% propylene ethylene copolymer (Adflex^{®} Q100F), and about 10 wt.% of a pigment master batch (Polybatch^{®} T93900), and outer layers formed from a mixture comprising about 48.5 wt.% EVA polymer (Escorene^{®} FL00218), about 48.5 wt.% PP elastomer (Vistamaxx^{®} 3980FL), about 2 wt.% slip/anti-block agent (Polybatch^{®} SAB1982VA) and about 1 wt.% polymer processing aid (AMF705HF).

Sample 3 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 97 wt.% EVA polymer (Escorene^{®} FL00218), about 2 wt.% slip/anti-block agent (Polybatch^{®} SAB1982V A) and about 1 wt.% polymer processing aid (AMF705HF).

Sample 4 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 96 wt.% EVA polymer (Escorene^{®} FL00218), about 3 wt.% slip/anti-block agent (Polybatch^{®} SAB1982V A) and about 1 wt.% polymer processing aid (AMF705HF).

Sample 5 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 65 wt.% PP elastomer (Vistamaxx^{®} 3980FL) and about 35 wt.% propylene ethylene copolymer (Adflex^{®} Q100F), and outer layers formed from a mixture comprising about 95 wt.% EVA polymer (Escorene^{®} FL00218), about 4 wt.% slip/anti-block agent (Polybatch^{®} SAB1982VA) and about 1 wt.% polymer processing aid (AMF705HF).

Sample 6 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 55 wt.% PP elastomer (Vistamaxx^{®} 3980FL), about 35 wt.% propylene ethylene copolymer (Adflex^{®} Q100F), and about 10 wt.% of a pigment master batch (Polybatch^{®} T93900), and outer layers formed from a mixture comprising about 97 wt.% EVA polymer (Escorene^{®} FL00218), about 2 wt.% slip/anti-block agent (Polybatch^{®} SAB1982VA) and about 1 wt.% polymer processing aid (AMF705HF).

Sample 7 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 55 wt.% PP elastomer (Vistamaxx^{®} 3980FL), about 35 wt.% propylene ethylene copolymer (Adflex^{®} Q100F), and about 10 wt.% of a pigment master batch (Polybatch^{®} T93900), and outer layers formed from a mixture comprising about 96 wt.% EVA polymer (Escorene^{®} FL00218), about 3 wt.% slip/anti-block agent (Polybatch^{®} SAB1982VA) and about 1 wt.% polymer processing aid (AMF705HF).

Sample 8 included a barrier layer formed from a mixture comprising about 85 wt.% amorphous polyamide (Selar^{®} PA3426) and about 15 wt.% ethylene ethyl acrylate maleic anhydride copolymer (Lotader^{®} 4720), tie layers formed from a neat maleic anhydride (MAH) grafted blend of ethylene propylene rubber (EPR) and polypropylene (PP) (Zelas^{®} MC721AP), inner layers formed from a mixture comprising about 55 wt.% PP elastomer (Vistamaxx^{®} 3980FL), about 35 wt.% propylene ethylene copolymer (Adflex^{®} Q100F), and about 10 wt.% of a pigment master batch (Polybatch^{®} T93900), and outer layers formed from a mixture comprising about 95 wt.% EVA polymer (Escorene^{®} FL00218), about 4 wt.% slip/anti-block agent (Polybatch^{®} SAB1982VA) and about 1 wt.% polymer processing aid (AMF705HF).

Samples 1 and 2 included outer layers formed from a mixture comprising EVA polymer, PP elastomer, and Polybatch^{®} SAB1982VA slip/anti-block agent. Samples 3-8 included outer layers formed from a mixture that is free of a non-polar polymer and comprising EVA polymer and Polybatch^{®} SAB1982VA slip/anti-block agent. As shown in Table 3, Samples 3 and 6, which included the outer layers formed from a mixture that is free of a non-polar polymer and comprising about 97 wt.% EVA polymer (Escorene^{®} FL00218), about 2 wt.% slip/anti-block agent (Polybatch^{®} SAB1982VA) and about 1 wt.% polymer processing aid (AMF705HF), had a significantly higher kinetic coefficient of friction when compared to Samples 1 and 2, which included the outer layers formed from a mixture containing a non-polar polymer and the same amount of slip/ani-block agent (Polybatch^{®} SAB1982VA.) Samples 4, 5, 7, 8, which included the outer layers formed from a mixture that is free of a non-polar polymer and comprising EVA polymer (Escorene^{®} FL00218), about 3 wt.% to about 4 wt.% slip/anti-block agent (Polybatch^{®} SAB1982VA), and about 1 wt.% polymer processing aid (AMF705HF), had a kinetic coefficient of friction that is comparable to Samples 1 and 2, which included the outer layers formed from a mixture containing a non-polar polymer and about 2 wt.% slip/ani-block agent (Polybatch^{®} SAB1982VA.)

Additional seven-layer film samples were prepared and tested for kinetic coefficient of friction according to ASTM D1894, blocking force according to ASTM D3354, and evaluated for adhesion strength of the closure members 222, 224. 226 (FIG. 3) to the outer layers of the film samples. Each of the film samples was configured to have an outer layer/inner layer/tie layer/barrier layer/tie layer/inner layer/outer layer configuration as shown in the embodiment of FIG. 1. The barrier layer, tie layers, and inner layers of each of the film samples were configured the same, wherein the barrier layer was formed from a mixture comprising about 85 wt.% amorphous polyamide (Selar^{®} PA3426) and about 15 wt.% ethylene ethyl acrylate maleic anhydride copolymer (Lotader^{®} 4720), the tie layers were formed from a neat MAH grafted blend of EPR and PP (Zelas^{®} MC721AP), and the inner layers were formed from a mixture comprising about 65 wt.% PP elastomer (Vistamaxx^{®} 3980FL) and about 35 wt.% propylene ethylene copolymer (Adflex^{®} Q100F).

**TABLE 4 Kinetic Coefficient of Friction, Blocking Force, and Lock'n Roll Member Adhesion Strength of Seven-layer Film Samples**

| | **Outer layers** | **Side*** | **CoF** | | | **Blocking Force (g/100cm²)** | **Lock'n Roll Member Adhesion Strength** | | |
|---|---|---|---|---|---|---|---|---|---|
| | | | **Day 2** | **Day 12** | **Day 30** | | **Initial** | **After 2 mins** | **After 20 hours** |
| **Sample 9** | 48.5wt.% Escorene^{®} FL00218, 48.5wt.% Vistamaxx^{®} 3980FL, 2wt.% Polybatch^{®} SAB1982VA, 1wt.% AMF705HF | out - out | 1.212 | 0.117 | 0.16 | 9.1 | Good | Strong | Strong |
| | | in - in | 1.177 | 0.117 | 0.19 | | | | |
| **Sample 10** | 94wt.% Escorene^{®} FL00218, wt.% Polybatch^{®} T02569, 2wt.% Polybatch^{®} M65 | out - out | 0.09 | 0.086 | 0.15 | 46.2 | Weak | Strong | Strong |
| | | in - in | 0.074 | 0.09 | 0.14 | | | | |
| **Sample 11** | 96wt% Escorene^{®} FL00218, 3wt.% Polybatch^{®} SAB1982VA, 1wt.% AMF705HF | out - out | 0.247 | 0.157 | 0.17 | 13 | Good | Weak | Peel off |
| | | in - in | 0.009 | 0.074 | 0.16 | | | | |
| **Sample 12** | 96wt.% Escorene^{®} FL00218, 3wt.% Polybatch^{®} T02569, 1wt.% Polybatch^{®}SAS | out - out | 0.082 | 0.094 | 0.17 | 73.7 | OK | Strong | Strong |
| | | in - in | 0.069 | 0.08 | 0.15 | | | | |
| **Sample 13** | 95wt.% Escorene^{®} FL00218, 4wt.% Polybatch^{®} T02569, 1wt.% Polybatch^{®} M65 | out - out | 0.086 | 0.096 | 0.14 | 76.1 | Good | Weak | Strong |
| | | in - in | 0.083 | 0.087 | 0.14 | | | | |
| **Sample 14** | 96.5wt.% Escorene^{®} FL00218, 2wt.% Polybatch^{®} T02569, 1.5wt.% Polybatch^{®} SAB1982VA | out - out | 0.091 | 0.084 | 0.17 | 9.6 | Weak | Weak | Peel off |
| | | in - in | 0.061 | 0.071 | 0.21 | | | | |

| | | | | | | | | | |
|---|---|---|---|---|---|---|---|---|---|
| * out-out: CoF test side for the film that is wound toward outside of a film roll In-in: CoF test side of the film that is wound toward inside of a film roll | | | | | | | | | |

Sample 9 included outer layers formed from a mixture comprising about 48.5 wt.% EVA polymer (Escorene^{®} FL00218), about 48.5 wt.% PP elastomer (Vistamaxx^{®} 3980FL), about 2 wt.% slip/anti-block agent (Polybatch^{®} SAB1982VA) and about 1 wt.% polymer processing aid (AMF705HF).

Sample 10 included outer layers formed from a mixture comprising about 94 wt.% EVA polymer (Escorene^{®} FL00218), about 4 wt.% slip agent (Polybatch^{®} T02569), and about 2 wt.% anti-block agent (Polybatch^{®} M65).

Sample 11 included outer layers formed from a mixture comprising about 96 wt.% EVA polymer (Escorene^{®} FL00218), about 3 wt.% slip/anti-block agent (Polybatch^{®} SAB1982V A) and about 1 wt.% polymer processing aid (AMF705HF).

Sample 12 included outer layers formed from a mixture comprising about 96 wt.% EVA polymer (Escorene^{®} FL00218), about 3 wt.% slip agent (Polybatch^{®} T02569) and about 1 wt.% anti-block agent (Polybatch^{®} SAS).

Sample 13 included outer layers formed from a mixture comprising about 95 wt.% EVA polymer (Escorene^{®} FL00218), about 4 wt.% slip agent (Polybatch^{®} T02569), and about 1 wt.% anti-block agent (Polybatch^{®} M65).

Sample 14 included outer layers formed from a mixture comprising about 95.5 wt.% EVA polymer (Escorene^{®} FL00218), about 2 wt.% slip agent (Polybatch^{®} T02569), and about 1.5 wt.% slip/anti-block agent (Polybatch^{®} SAB1982VA).

As shown in Table 4, Sample 10 including outer layers formed from a mixture comprising about 94 wt.% EVA polymer (Escorene^{®} FL00218), about 4 wt.% slip agent (Polybatch^{®} T02569), and about 2 wt.% anti-block agent (Polybatch^{®} SAS), had excellent kinetic coefficient of friction, blocking force, and adhesion properties for making an ostomy pouch.

All patents referred to herein, are hereby incorporated herein in their entirety, by reference, whether or not specifically indicated as such within the text of this disclosure.

In the present disclosure, the words "a" or "an" are to be taken to include both the singular and the plural. Conversely, any reference to plural items shall, where appropriate, include the singular.

From the foregoing it will be observed that numerous modifications and variations can be effectuated without departing from the true spirit and scope of the novel concepts of the present invention. It is to be understood that no limitation with respect to the specific embodiments illustrated is intended or should be inferred. The disclosure is intended to cover by the appended claims all such modifications as fall within the scope of the claims.

Furthermore, one or more of the following numbered clauses may describe and relate to further aspects or features within the context of the present teaching.
1. A multilayer film for an ostomy appliance, comprising: a barrier layer, the barrier layer being substantially impermeable to malodor causing compounds; and first and second outer layers, each of the first and second outer layers formed from a polymeric mixture comprising about 90 wt.% to about 99 wt.% of a polar polymer and about 1 wt.% to about 10 wt.% of a slip agent and/or an anti -block agent, wherein the polymeric mixture is free of a non-polar polymer, wherein the barrier layer is arranged between the first and second outer layers.
2. The multilayer film of clause 1, wherein the multilayer film has a kinetic coefficient of friction of about 0.05 to about 0.30 when measured according to ASTM D1894.
3. The multilayer film of clause 1, wherein the multilayer film has a kinetic coefficient of friction of about 0.06 to about 0.28 when measured according to ASTM D1894.
4. The multilayer film of clause 1. wherein the multilayer film has a kinetic coefficient of friction of about 0.08 to about 0.25 when measured according to ASTM D1894.
5. The multilayer film of any of clauses 1-4, wherein the multilayer film has a blocking force below about IOOg/IOOcm 2 when measured according to ASTM D3354.
6. The multilayer film of any of clauses 1-4, wherein the multilayer film has a blocking force below about 80g/100cm 2 when measured according to ASTM D3354.
7. The multilayer film of clause 1, wherein the multilayer film has a kinetic coefficient of friction of about 0.06 to about 0.20 when measured according to ASTM DI 894 and a blocking force below about 70g/100cm 2 when measured according to ASTM D3354.
8. The multilayer film of any of clauses 1-6, wherein each of the first and second outer layers is formed from a polymeric mixture comprising about 92 wt.% to about 97 wt.% of a polar polymer and about 3 wt.% to about 8 wt.% of a slip agent and/or an anti-block agent.
9. The multilayer film of any of clauses 1-6. wherein each of the first and second outer layers is formed from a polymeric mixture comprising about 93 wt.% to about 96 wt.% of a polar polymer and about 4 wt.% to about 7 wt.% of a slip agent and/or an anti-block agent.
10. The multilayer film of any of clauses 1-8, wherein the polar polymer is selected from the group consisting of ethylene vinyl acetate (EVA) copolymer, ethylene methyl acrylate copolymer, ethylene acry lic acid copolymer, ethylene methyl acrylic acid copolymer, and polylactic acid homopolymer.
11. The multilayer film of any of clauses 1-9, wherein the slip agent is selected from the group consisting of oleamide, erucamide, stearamide, behenamide. oley/palmitamide, stearyl erucamide, ethylene bis-steramide, ethylene bis-oleamide, and the blends thereof.
12. The multilayer film of any of clauses 1-10, wherein the anti-block agent is selected from the group consisting of silica, talc, kaolin, mica, calcium carbonate, paraffinic waxes, ethylene and propylene oxide, fatty 7 acid soaps, fatty 7 acid amides, silicones.
13. The multilayer film of clause 1, wherein each of the first and second outer layers is formed from a polymeric mixture comprising about 93 wt.% to about 95 wt.% of an EVA copolymer, about 3 wt.% to about 5 wt.% of a slip agent, and about 1 wt.% to about 3 wt.% of an anti-block agent, wherein the multilayer film has a kinetic coefficient of friction of about 0.06 to about 0.20 when measured according to ASTM D1894 and a blocking force below- about 70g/100cm 2 when measured according to ASTM D3354.
14. The multilayer film of clause 1, wherein each of the first and second outer layers is formed from a polymeric mixture comprising about 94 wt.% of an EVA copolymer, about 4 wt.% of a slip agent, and about 2 wt.% of an anti -block agent, wherein the multilayer film has a kinetic coefficient of friction of about 0.06 to about 0.20 when measured according to ASTM DI 894 and a blocking force below about 70g/100cm 2 when measured according to ASTM D3354.
15. The multilayer film of any of clauses 1-14, wherein the barrier layer is formed from a mixture comprising about 65 wt.% to about 100 wt.% of an amorphous polyamide and about 0 wt.% to about 35 wt.% of a maleic anhydride modified olefin or an epoxy modified olefin.
16. The multilayer film of any of clauses 1-15, wherein the multilayer film has an outer layer/inner layer/tie layer/barrier layer/tie layer/inner layer/outer layer construction.
17. The multilayer film of clause 16, wherein the tie layers are formed from a maleic anhydride grafted resin, wherein the resin is one or more of an ethylenebased copolymer, a propylene-based copolymer, an ethylene-octene polymer and a styrene block copolymer, and wherein the inner layers are formed from an ethylene propylene copolymer (polypropylene elastomer) based resin, an ethylene-octene based resin or blends thereof.
18. The multilayer film of any of clauses 16-17, wherein the multilayer film is symmetrical about the barrier layer.
19. The multilayer film of any of clauses 16-18, wherein the multilayer film has an Elmendorf tear strength in machine direction measured by ASTM DI 9222-09 of at least about 200g/mil.
20. An ostomy pouch formed of the multilayer film of any of clauses 1-19, comprising: two side walls, wherein each of the side walls is formed of the multilayer film of any of clauses 1-19; a stoma-receiving opening on one of the side walls; and wherein the two side walls are heat sealed together along peripheral edges of the side walls.

## Claims

1. A multilayer film for an ostomy appliance, comprising:
a barrier layer, the barrier layer being substantially impermeable to malodor causing compounds; and
first and second outer layers, each of the first and second outer layers formed from a polymeric mixture comprising about 90 wt.% to about 99 wt.% of a polar polymer and about 1 wt.% to about 10 wt.% of a slip agent and/or an anti-block agent, wherein the polymeric mixture is free of a non-polar polymer,
wherein the barrier layer is arranged between the first and second outer layers.

2. The multilayer film of claim 1, wherein the multilayer film has a kinetic coefficient of friction of about 0.05 to about 0.30 when measured according to ASTM D1894.

3. The multilayer film of any of claims 1-2, wherein the multilayer film has a blocking force below about 100g/100cm² when measured according to ASTM D3354.

4. The multilayer film of claim 1, wherein the multilayer film has a kinetic coefficient of friction of about 0.06 to about 0.20 when measured according to ASTM D1894 and a blocking force below about 70g/100cm² when measured according to ASTM D3354.

5. The multilayer film of any of claims 1-4 wherein each of the first and second outer layers is formed from a polymeric mixture comprising about 92 wt.% to about 97 wt.% of a polar polymer and about 3 wt.% to about 8 wt.% of a slip agent and/or an anti-block agent.

6. The multilayer film of any of claims 1-4, wherein each of the first and second outer layers is formed from a polymeric mixture comprising about 93 wt.% to about 96 wt.% of a polar polymer and about 4 wt.% to about 7 wt.% of a slip agent and/or an anti-block agent.

7. The multilayer film of any of claims 1-6, wherein the polar polymer is selected from the group consisting of ethylene vinyl acetate (EVA) copolymer, ethylene methyl acrylate copolymer, ethylene acrylic acid copolymer, ethylene methyl acrylic acid copolymer, and polylactic acid homopolymer.

8. The multilayer film of any of claims 1-7, wherein the slip agent is selected from the group consisting of oleamide, erucamide, stearamide, behenamide, oley/palmitamide, stearyl erucamide, ethylene bis-steramide, ethylene bis-oleamide, and the blends thereof.

9. The multilayer film of any of claims 1-8, wherein the anti-block agent is selected from the group consisting of silica, talc, kaolin, mica, calcium carbonate, paraffinic waxes, ethylene and propylene oxide, fatty acid soaps, fatty acid amides, silicones.

10. The multilayer film of any of claims 1-9, wherein the barrier layer is formed from a mixture comprising about 65 wt.% to about 100 wt.% of an amorphous polyamide and about 0 wt.% to about 35 wt.% of a maleic anhydride modified olefin or an epoxy modified olefin.

11. The multilayer film of any of claims 1-10, wherein the multilayer film has an outer layer/inner layer/tie layer/barrier layer/tie layer/inner layer/outer layer construction.

12. The multilayer film of claim 11, wherein the tie layers are formed from a maleic anhydride grafted resin, wherein the resin is one or more of an ethylene-based copolymer, a propylene-based copolymer, an ethylene-octene polymer and a styrene block copolymer, and wherein the inner layers are formed from an ethylene propylene copolymer (polypropylene elastomer) based resin, an ethylene-octene based resin or blends thereof.

13. The multilayer film of any of claims 11-12, wherein the multilayer film is symmetrical about the barrier layer.

14. The multilayer film of any of claims 11-13, wherein the multilayer film has an Elmendorf tear strength in machine direction measured by ASTM D19222-09 of at least about 200g/0.0254mm (200g/mil).

15. An ostomy pouch formed of the multilayer film of any of claims 1-14, comprising:
two side walls, wherein each of the side walls is formed of the multilayer film of any of claims 1-14;
a stoma-receiving opening on one of the side walls; and
wherein the two side walls are heat sealed together along peripheral edges of the side walls.
